(19) Europäisches Patentamt European Patent Office Office européen des brevets

(11) **EP 4 631 538 A1**

(12) **EUROPEAN PATENT APPLICATION**

(43) Date of publication:
**15.10.2025 Bulletin 2025/42**

(21) Application number: **24382364.8**

(22) Date of filing: **09.04.2024**

(51) International Patent Classification (IPC):
*A61L 27/26* (2006.01)    *A61L 27/38* (2006.01)
*A61L 27/50* (2006.01)    *A61L 27/58* (2006.01)
*B33Y 70/00* (2020.01)    *B33Y 80/00* (2015.01)

(52) Cooperative Patent Classification (CPC):
(C-Sets available)
**A61L 27/3804; A61L 27/26; A61L 27/3834;
A61L 27/3843; A61L 27/50; A61L 27/58;
B33Y 70/00; B33Y 80/00;** A61L 2400/06;
A61L 2430/02; A61L 2430/34      (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Universidad de Granada**
**18071 Granada (ES)**
• **Universidad De Jaen**
**23071 Jaen (ES)**
• **Universidade da Coruña**
**15071 A Coruña (ES)**

(72) Inventors:
• **MARCHAL CORRALES, Juan Antonio**
**18016 Granada (ES)**
• **PLEGUEZUELOS BELTRÁN, Paula**
**18016 Granada (ES)**
• **LÓPEZ RUIZ, Elena**
**23071 Jaén (ES)**
• **NIETO GARCÍA, Daniel**
**15071 A Coruña (ES)**

(74) Representative: **Hoffmann Eitle**
**Hoffmann Eitle S.L.U.
Paseo de la Castellana 140, 3a planta
Edificio LIMA
28046 Madrid (ES)**

(54) **NOVEL FIBRINOGEN-BASED BIOINKS FOR WOUND HEALING AND TISSUE REGENERATION**

(57) The present invention relates to a bioink formulation comprising fibrinogen and a glycosaminoglycans (GAGs)/collagen (Col) matrix, preferably comprising human cells, preferably human mesenchymal stem cells (hMSCs), keratinocytes and/or human dermal fibroblasts (hDFs), and its use in the treatment of tissue or bone injuries or damages, specifically skin tissue injuries or damages.

A

Control (n=8)   Autograft (n=8)   FibD (n=8)   FibD + hMSCs (n=8)

16 mm

① ② ③ ④ ⑤

**(Cont. next page)**

EP 4 631 538 A1

(52) Cooperative Patent Classification (CPC): (Cont.)

C-Sets
**A61L 27/26, C08L 5/00;**
**A61L 27/26, C08L 5/08;**
**A61L 27/26, C08L 89/00;**
**A61L 27/26, C08L 89/06**

**Description**

## TECHNICAL FIELD

The present invention relates to a bioink formulation comprising fibrinogen and a glycosaminoglycans (GAGs)/-collagen (Col) matrix, preferably comprising human cells, preferably human mesenchymal stem cells (hMSCs), keratinocytes and/or human dermal fibroblasts (hDFs), and its use in the treatment of tissue or bone injuries or damages, specifically skin tissue injuries or damages.

## BACKGROUND OF THE INVENTION

Skin is one of the most important defense mechanisms in the body, protecting it against pathogens and chemical and physical hazards. The skin is composed of three main layers: the top layer is the epidermis, the middle and thicker layer is the dermis, and the subcutaneous tissue is the hypodermis. Skin wounds or injuries are breaks in the skin tissue, which may result from different causes, such as physical or chemical traumas (including burns or removal of skin during surgery), genetic irregularities, or skin diseases, such as epidermolysis bullosa, perianal fistulae or chronic wounds (like diabetic foot ulcers, pressure ulcers, and leg ulcers). Shallow injuries can usually be regenerated naturally by the skin's self-healing functions, but in deep wounds, the skin's regenerative components are destroyed, therefore requiring therapeutic interventions to facilitate wound healing.

Traditionally, the gold standard in clinical practice for treating deep wounds has been the use of autologous skin grafts (autografts). Despite their effectiveness, autografts present important limitations, such as the unavailability of sufficient donor skin in cases like major burn patients, and the creation of a secondary wound for the patient when harvesting said donor skin. Other grafting options include the culture of cells harvested from a small donor site to amplify their number, obtaining cultured epithelial autografts (CEAs), which require weeks to become available, or the temporary use of allografts or xenografts, which have the risk of diseases transmission and immune rejection.

On the other hand, thanks to the advances in regenerative medicine and tissue engineering, different biofabrication methods, such as 3D bioprinting or electrospinning),have been developed for the production of skin substitutes, which are defined as a heterogeneous group of substances, including hydrogels, cell suspensions, films, 3D scaffolds or cell sheets, intended to cover the wound and act as a barrier to avoid infections and fluid loss, promote wound healing, and reduce pain. Numerous cellular and acellular skin substitutes are commercially available for clinical use.

The biofabrication processes generally make use of biomaterial inks, which are acellular formulations containing biologically active components or molecules that can be subsequently seeded with cells, or bioinks, i.e., cell-based formulations that may also contain biomaterials and other biologically active components. To manufacture skin substitutes, bioinks have been formulated with various natural and synthetic polymeric biomaterials that assemble into three-dimensional networks with a structure similar to the natural ECM, where cells can adhere, proliferate, differentiate, and promote skin regeneration. These biomaterials ought to have good mechanical properties, be biocompatible and biodegradable, be capable of maintaining humidity, and have enough porosity to allow cell migration and proliferation and the transport of nutrients and metabolic wastes. Some examples of biomaterials used for skin substitutes are collagen, fibrin, alginate, gelatin, glycosaminoglycans (GAGs), or chitosan.

Among them, fibrin is highly biocompatible and biodegradable, and plays a vital role in wound healing and hemostasis. It is obtained during the coagulation cascade, when fibrinogen is converted into a fibrin clot by the action of thrombin. Fibrin promotes cell migration toward the wound site and forms a matrix where cells can proliferate and promote tissue regeneration. Moreover, it has been reported to reduce scar formation, and can be isolated from the patient's blood, avoiding immunogenicity.

GAGs are a group of polysaccharides, including hyaluronic acid (HA), heparan sulfate, dermatan sulfate, chondroitin sulfate, and keratan sulfate, which play crucial roles in all stages of wound healing. HA, a major ECM component, is the skin's predominant GAG. It is biocompatible and biodegradable and has anti-inflammatory and mucoadhesive properties. Furthermore, it has a high-water retention capability, which defines skin elasticity, and it inhibits scar tissue formation.

One biofabrication technique disclosed in the prior art for application in skin regeneration is the use of skin sprays, which present several advantages for the delivery of bioinks or biomaterial inks to the wound bed: the possibility to treat large wounds or areas with unfavorable topography, in a rapid manner, with much more facility, and obtaining a homogeneous distribution of the sprayed material.

Acellular sprays generally consist of biomaterial inks that, when sprayed over the wound bed, create thin films or dressings covering the wounds, protecting them against TEWL and infections, and promoting the wound healing process. There are several acellular spray products that have been commercialized for decades now, the majority of them based on fibrin due to its hemostatic properties: for example, TISSEEL® and ARTISS® by Baxter [1,2], VISTASEAL™ by Johnson & Johnson [3], or Vivostat's Fibrin Sealant [4]. Other biomaterials that have been studied for acellular sprays are alginate [5], gelatin [6], chitosan [7], HA [8], and peptin [9]. WO2021015631 describes multilayered nanofibre compositions with bioactives for wound healing management. Upon exposure to moisture or on wet skin, each layer of the nanofibre composition dissolves at a desired controlled rate to release the bioactive or a non-dissolvable layer may function as a sacrificial skin layer. Disolvable polymers used can be among others Glycosaminoglycans (GAG's) like hyaluronic acid and the slow degrading polymer can be a slow-degradable collagen.

On the other hand, cellular sprays apply different types of cell suspensions or bioinks. For *in vivo* studies, autologous epidermal cells (normally fibroblasts or keratinocytes) are mainly used, although allogeneic cells have also been studied. Once sprayed over the wound bed, epidermal cells can remain viable and proliferate, promoting re-epithelialization. Cell spray autografting consists in taking a biopsy of the patient's undamaged skin, digesting said biopsy, and obtaining a solution of autologous epidermal cells that can be sprayed immediately over the wound.

In contrast to traditional sheet autografts, cell sprays only require a much smaller donor area and offer a cost-effective, simple, and immediate treatment for the patient. Moreover, the harvested autologous cell can also be cultured to expand their number before being sprayed. There are many reports of cellular sprays being studied *in vitro* [10,11], in animals [12,13] and in patients [14-17], for burns and wound healing applications, typically using airbrushes or syringes with spray nozzles as the spraying devices. However, few cell spray products have been approved for commercialization and use in clinical practice. The ReCell® kit by Avita Medical was the first "spray-on-skin" treatment approved by the U.S. Food and Drug Administration (FDA) in 2018, and it employs an enzyme solution to isolate autologous epidermal cells from a small biopsy of the patient's healthy skin, which are then suspended in a lactate solution and sprayed over the wound using a syringe with a spray nozzle [18]. Similarly, RenovaCare developed another enzymatic isolation technique to obtain a solution of autologous epidermal cells from a biopsy of the patient, called the "CellMist Solution", which is delivered through their SkinGun, an electronically controlled pneumatic spray device. However, this product has not received approval for commercialization yet [19].

Normally, cell suspensions can run off when sprayed over the body's convex structures, reducing the number of cells that stay in contact with the wound bed. To avoid this, cell suspensions can be sprayed in combination with biomaterials, such as fibrin, to ensure that the solution stays in place and cells adhere to the wound.

For example, US2006172008 A1 discloses porous freeze-dried fibrin matrices for in vitro and in vivo cell growth and tissue regeneration in which fibrin and hyaluronic acid are used to prepare the matrix.

US2017304600A1 discloses a device and methods suitable for producing a cellular spray of cells for covering and growing cells on a damaged tissue, such as a skin wound. The cellular spray comprises tissue regenerating cells in a physiological solution with electrolytes which can be applied to a damaged tissue. However, the physiological solution does not have the necessary mechanical properties for providing a suitable porosity and viscosity for cell migration and proliferation and the transports of nutrients and metabolic wastes, all of which prolongs cell viability. Furthermore, the physiological solution does not support the cell adherence for a prolonged time on the damaged tissue.

As mentioned above, despite their advantages, the translation of cell sprays to clinical practice is still limited and very few products have been approved for clinical use to date. There is therefore a need for providing novel bioinks for application in the treatment of skin lesions that are suitable for easy application, biocompatible and provide optimal wound healing properties.

## SUMMARY OF THE INVENTION

The present invention therefore relates in one aspect to a bioink formulation comprising fibrinogen and a glycosaminoglycans (GAGs)/collagen (Col) matrix, wherein the GAGs/Col matrix comprises at least

a) 60 - 75% (w/w) hyaluronic acid, and
b) 5 - 20% (w/w) sulphated GAGs, and
c) 1 - 5% (w/w) of collagen.

In one embodiment of the bioink formulation of present invention, the bioink formulation further comprises human cells, preferably human mesenchymal stem cells (hMSCs), keratinocytes and/or human dermal fibroblasts (hDFs).

In one embodiment of the bioink formulation of present invention the amount of fibrinogen in the formulation is between 1 and 100 mg/ml.

In one embodiment of the bioink formulation of present invention the amount of fibrinogen is about 10 mg/ml.

In one embodiment of the bioink formulation of present invention the amount of fibrinogen in the formulation is between 0.1 and 10 % (w/v).

In one embodiment of the bioink formulation of present invention the amount of fibrinogen is about 1% (w/v).

In one embodiment of the bioink formulation of present invention the amount of GAGs/Col matrix in the formulation is between 0.1 and 2.5% (w/v). In one embodiment of the bioink formulation of present invention the amount of GAGs/Col matrix in the formulation is between 1 and 25 mg/ml.

In one embodiment of the bioink formulation of present invention the amount of hyaluronic acid in the GAGs/Col matrix is between 65 and 70% (w/w).

In one embodiment of the bioink formulation of present invention the amount of collagen in the GAGs/Col matrix is between 5 and 15% (w/w), preferably between 8 and 12% (w/w).

In one embodiment of the bioink formulation of present invention the sulphated GAGs are selected from dermatan sulphate and/or chondroitin sulphate.

In one embodiment of the bioink formulation of present invention the amount of sulphated GAGs in the GAGs/Col matrix is between 10 and 15% (w/w).

In a further aspect the present invention relates to the bioink formulation as described herein for use in the treatment of tissue injuries or damages, comprising a step of administering the bioink to the tissue.

In a preferred embodiment of the tissue is skin.

In one embodiment the tissue injuries or damages are skin injuries or damages, preferably selected from burns, wounds, chronic wounds, such as for example diabetic foot ulcers, pressure ulcers, venous leg ulcers, other skin injuries, such as psoriasis, dermatitis, acne, vitiligo.

In one embodiment, wherein the skin injury or damage is a wound, the method comprises a step of administering the bioink onto the wound during or after surgery for wound sealing.

In one embodiment of the bioink formulation for use in the treatment of tissue injuries or damages the step of administering the bioink formulation to said tissue induces hemostasis.

In one embodiment of said aspect the bioink formulation further comprises hydroxyapatite nanoparticles.

In another aspect the present invention relates to the use of the bioink formulation as described herein as a tissue adhesive or glue for surgery wounds/wound sealing.

In a further aspect the present invention relates to the use of the bioink of present invention for bone healing. In a preferred embodiment of this aspect the bioink formulation further comprises hydroxyapatite nanoparticles.

In a further aspect the present invention relates to a method for treating diseased or injured tissue, the method comprising implanting/providing to the site of disease or injury a bioink formulation as described herein. In a preferred embodiment of the method the tissue is skin.

In a further aspect the present invention relates to the bioink formulation as described herein for use in the treatment of wrinkles or scars, or for the healing of the skin following aesthetic treatments, such as for example microneedling, laser, capacitive resistive monopolar radiofrequency, ultrasounds or peeling treatments.

## BRIEF DESCRIPTION OF FIGURES

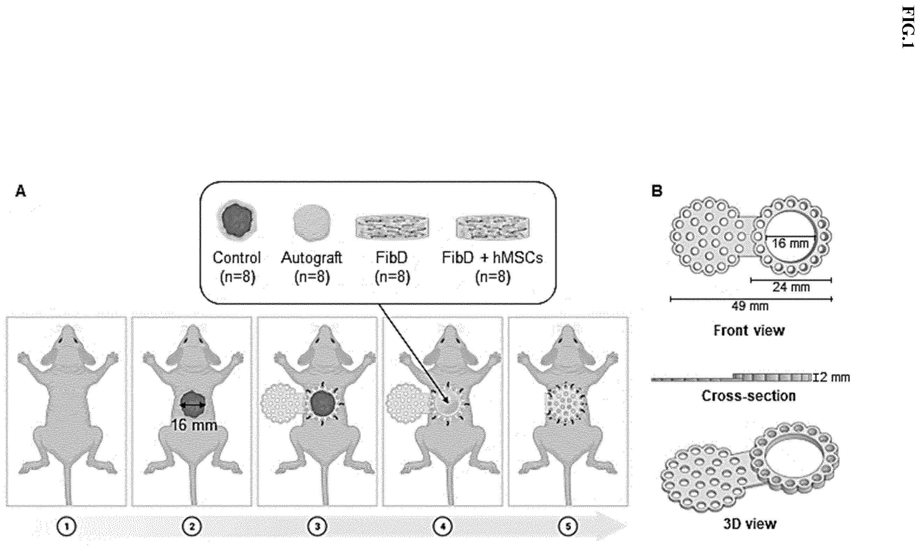

Figure 1: (A) In vivo excisional wound healing assay surgical protocol. (B) b-TPUe splint design. Figure created with BioRender.com.

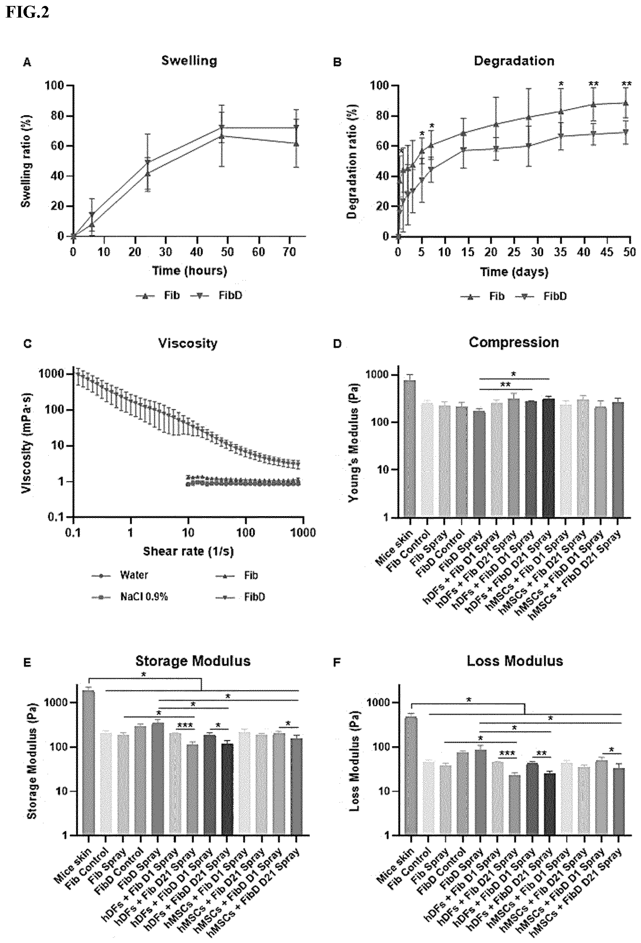

Figure 2: Physicochemical, rheological and mechanical characterization. (A) Swelling and (B) Degradation ratio of Fib and FibD hydrogels. (C) Viscosity of distilled water, 0.9% NaCl, and Fib and FibD inks. (D) Young's Modulus; and Viscoelastic moduli (E, Storage Modulus; F, Loss Modulus), of mice skin and hydrogels. Statistical significance: *$p < 0.05$; **$p < 0.01$; ***$p < 0.005$.

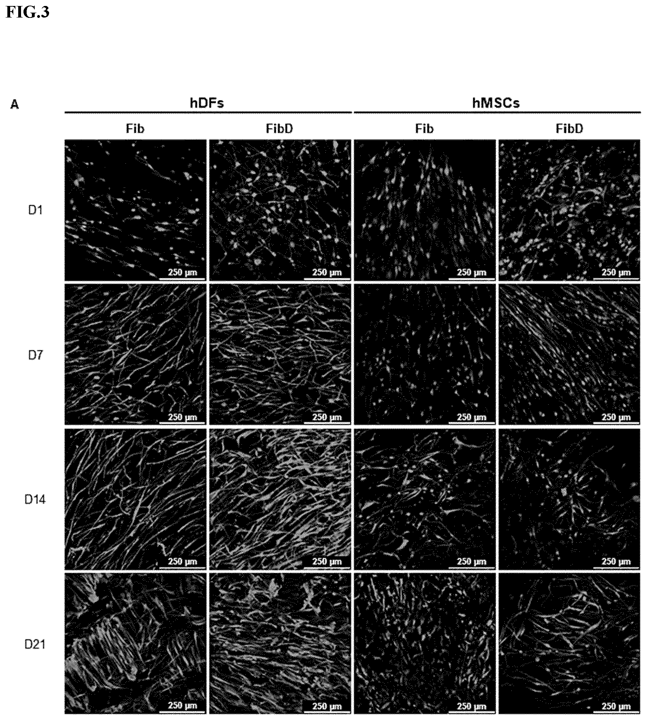

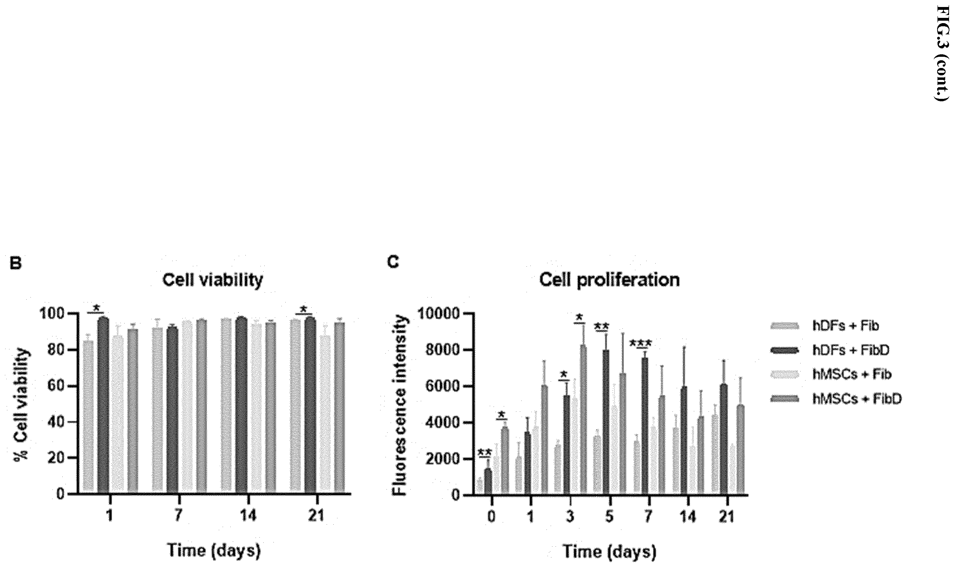

Figure 3: Cell viability and proliferation of hDFs and hMSCs in the sprayed Fib and FibD hydrogels. (A) Representative confocal images at days 1, 7, 14 and 21, with live cells stained in green (calcein AM) and dead cells stained in red (EthD-I); scale bars: 250 μm. (B) Cell viability (%), at days 1, 7, 14 and 21. (C) Cell proliferation, measured as fluorescence intensity (AU), at days 0, 1, 3, 5, 7, 14 and 21. Statistical significance: *$p < 0.05$, **$p < 0.01$, ***$p < 0.005$.

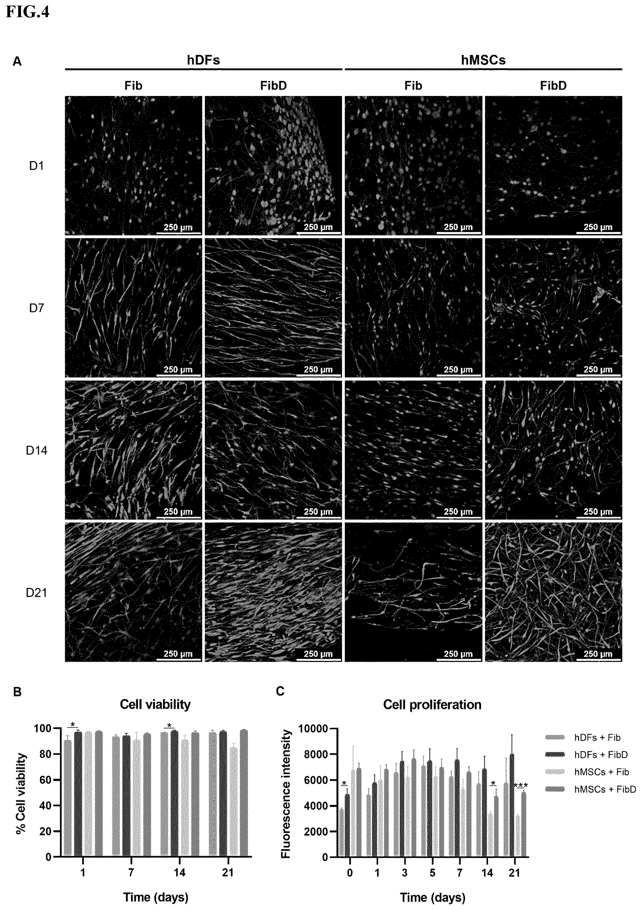

Figure 4: Cell viability and proliferation of hDFs and hMSCs in the pipetted (Control) Fib and FibD hydrogels. (A) Representative confocal images at days 1, 7, 14, and 21, with live cells stained in green (calcein AM) and dead cells stained in red (EthD-I); scale bars: 250 μm. (B) Cell viability (%), at days 1, 7, 14, and 21. (C) Cell proliferation, measured as fluorescence intensity (AU), at days 0, 1, 3, 5, 7, 14, and 21. Statistical significance: *$p < 0.05$, **$p < 0.01$, ***$p < 0.005$.

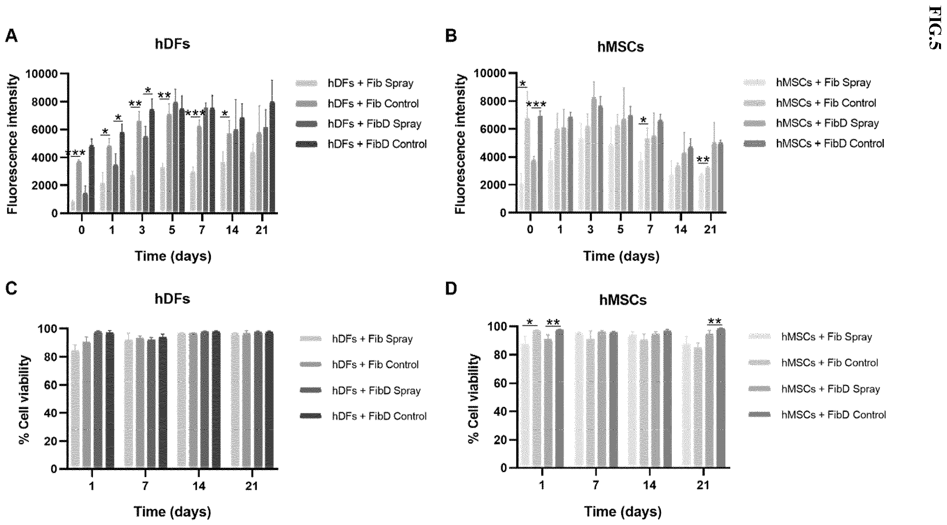

Figure 5: (A) Cell proliferation, measured as fluorescence intensity (AU), of hDFs in the pipetted (Control) and sprayed Fib and FibD hydrogels, at days 0, 1, 3, 5, 7, 14, and 21. (B) Cell proliferation, measured as fluorescence intensity (AU), of hMSCs in the pipetted (Control) and sprayed Fib and FibD hydrogels, at days 0, 1, 3, 5, 7, 14, and 21. (C) Cell viability (%) of hDFs in the pipetted (Control) and sprayed Fib and FibD hydrogels, at days 1, 7, 14, and 21. (D) Cell viability (%) of hMSCs in the pipetted (Control) and sprayed Fib and FibD hydrogels, at days 1, 7, 14, and 21. Statistical significance: *p < 0.05, **p < 0.01, ***p < 0.005.

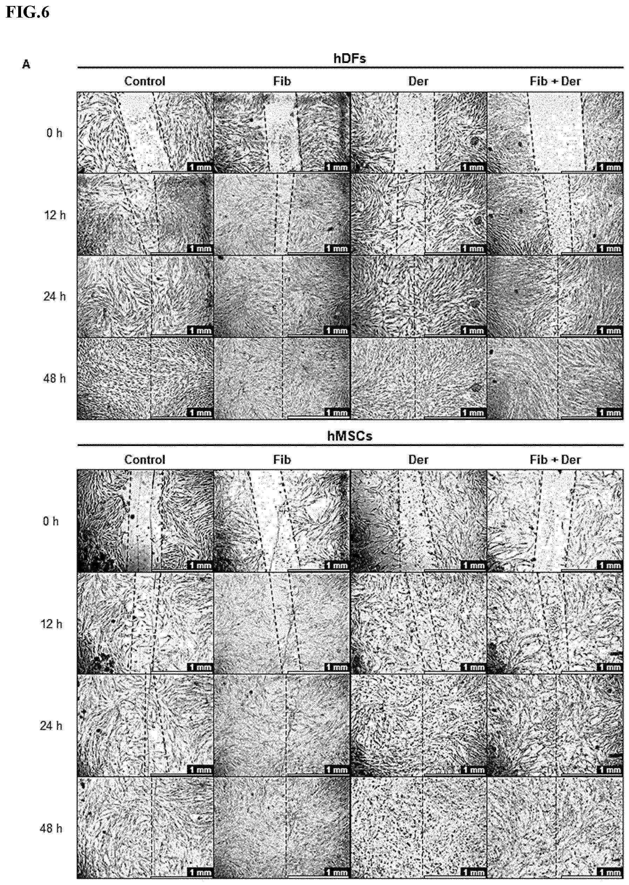

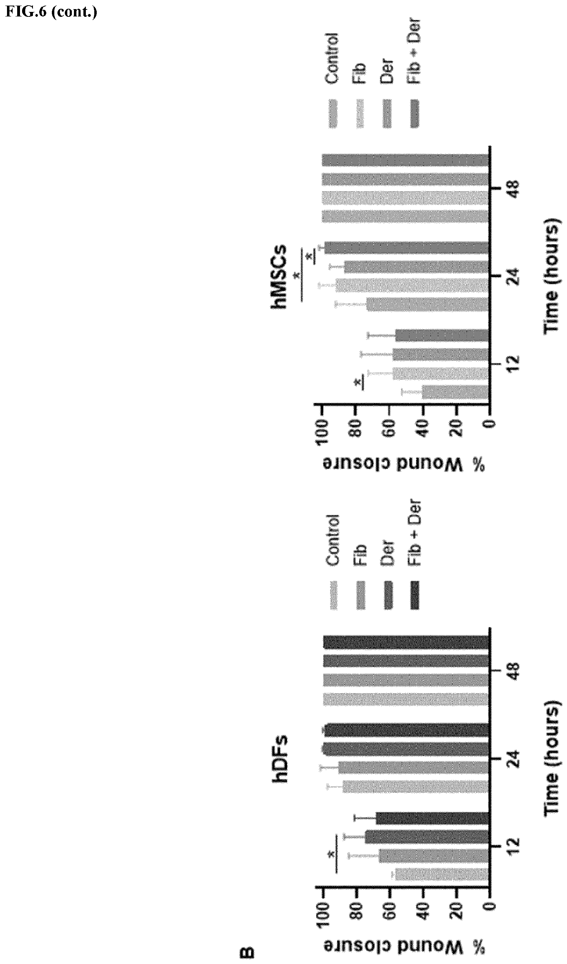

Figure 6: Scratch wound healing assay of hDFs and hMSCs treated with normal culture media (Control) or culture media supplemented with Fib, Der, or Fib + Der. (A) Representative microscope images (scale bars: 1mm); and (B) % of wound closure for hDFs and hMSCs after 12, 24 and 48 hours. Statistical significance: *p < 0.05.

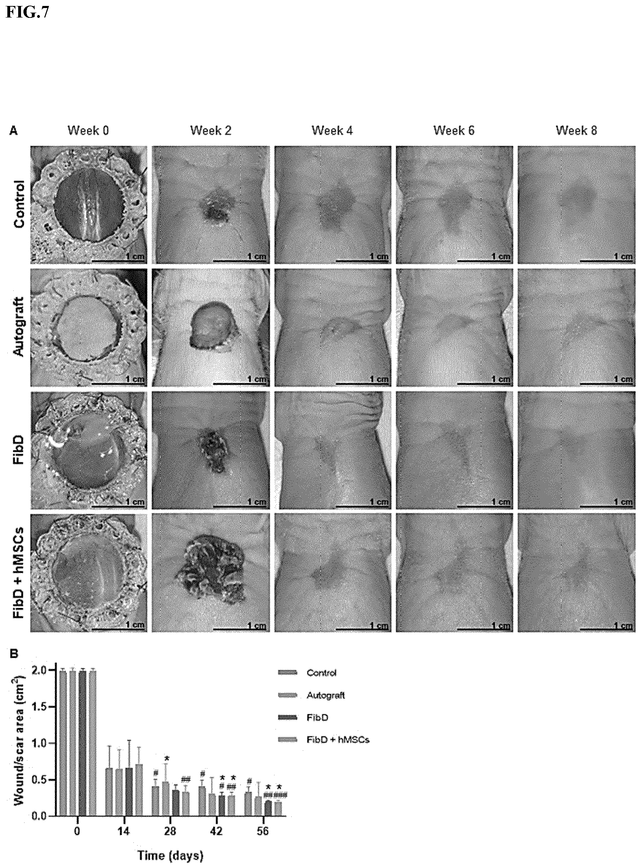

Figure 7: (A) Macroscopic images of wound healing process over time (weeks 0, 2, 4, 6 and 8) for non-treated mice (Control) or mice treated with Autograft, FibD, or FibD + hMSCs; scale bars: 1 cm. (B) Quantitative evaluation of wound/scar area through time for all groups. Statistical significance compared to Control: *p < 0.05. Statistical significance compared to day 14: #p < 0.05; ##p < 0.01; ###p < 0.005.

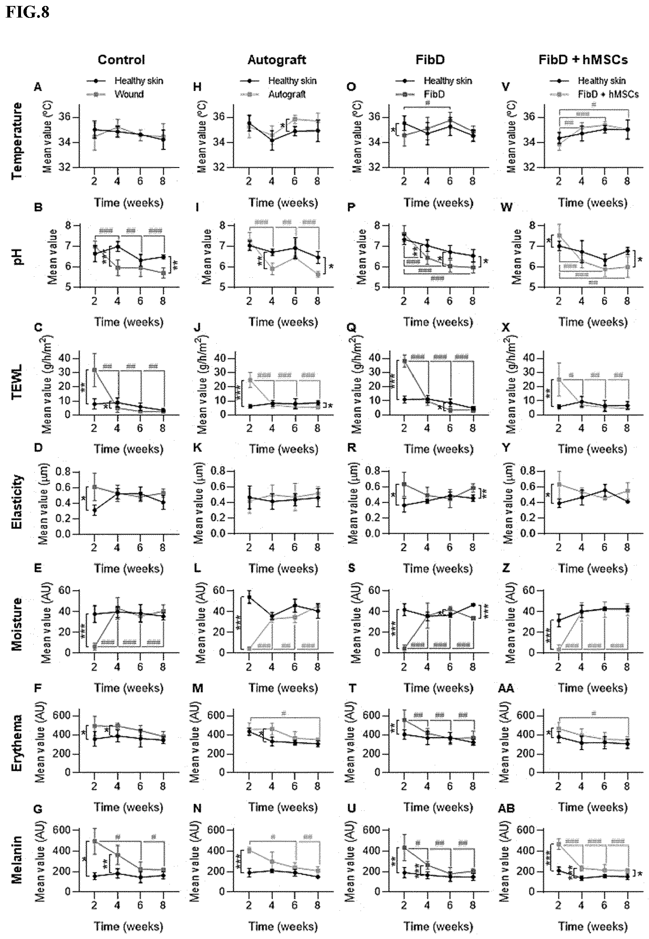

Figure 8: Analysis of homeostasis parameters per week and group. Graphics show results of regenerated wounds/scars against healthy skin for each group of treatment: Control (A, B, C, D, E, F and G), Autograft (H, I, J, K, L, M and N), FibD (O, P, Q, R, S, T and U), and FibD + hMSCs (V, W, X, Y, Z, AA and AB). Results per week were calculated as the mean value of all mice measured at each time of study (n week 2, 4, 6, 8 = 8, 8, 4, 4). Statistical significance compared to healthy skin: *p < 0.05; **p < 0.01; ***p < 0.005. Statistical significance compared to day 14: #p < 0.05; ##p < 0.01; ###p < 0.005.

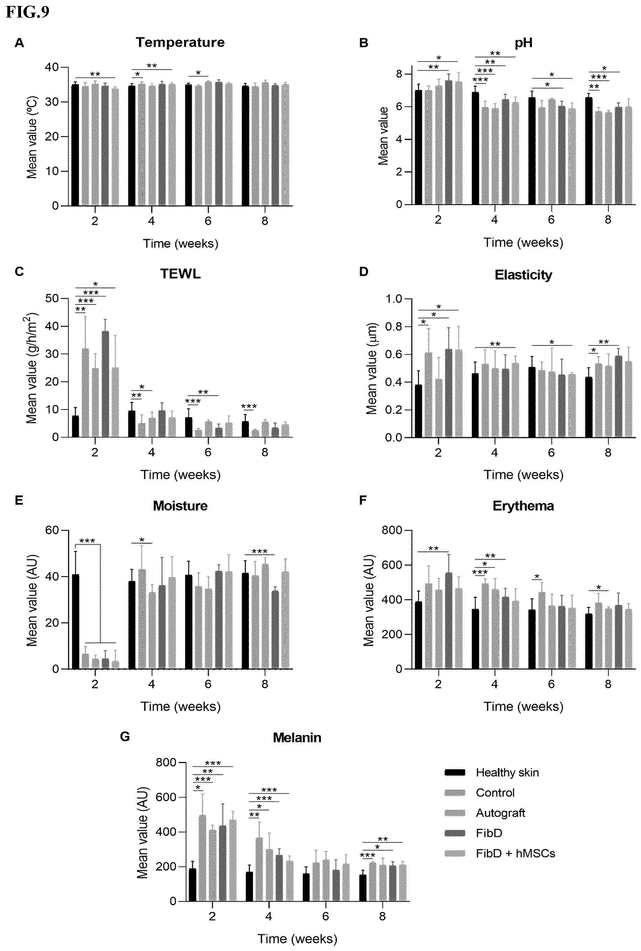

Figure 9: Analysis of homeostasis per parameter: (A) Temperature; (B) pH; (C) TEWL; (D) Elasticity; (E) Moisture; (F) Erythema; and (G) Melanin. Graphics show the regenerated wounds/scars results for each treatment group (Control, Autograft, FibD, and FibD + hMSCs) against healthy skin. Results per week were calculated as the mean value of all mice measured at each time of study: Control, Autograft, FibD, and FibD + hMSCs (n week 2, 4, 6, 8 = 8, 8, 4, 4, respectively); Healthy skin (n week 2, 4, 6, 8 = 32, 32, 16, 16, respectively). Statistical significance compared to healthy skin: *p < 0.05, **p < 0.01, ***p < 0.005.

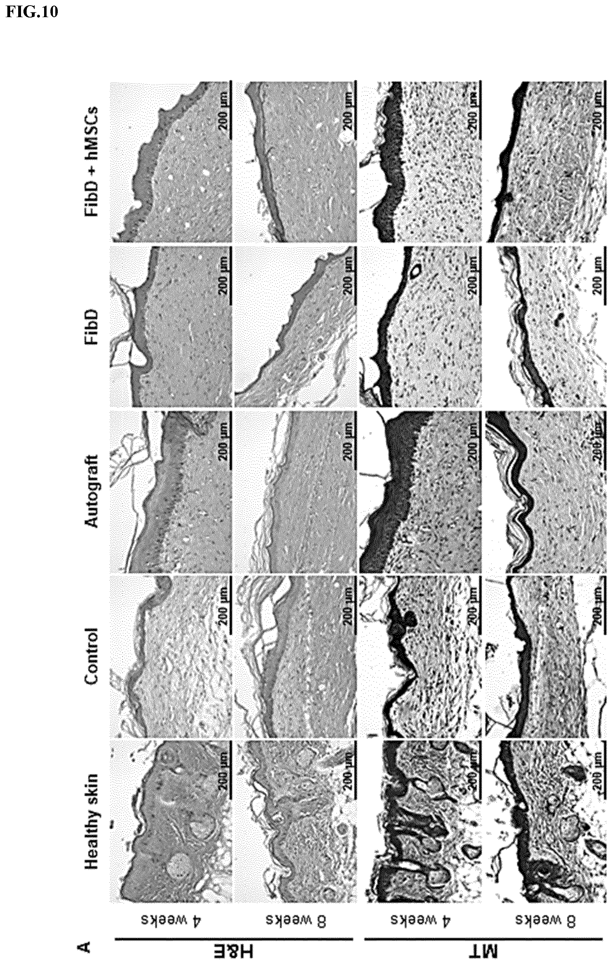

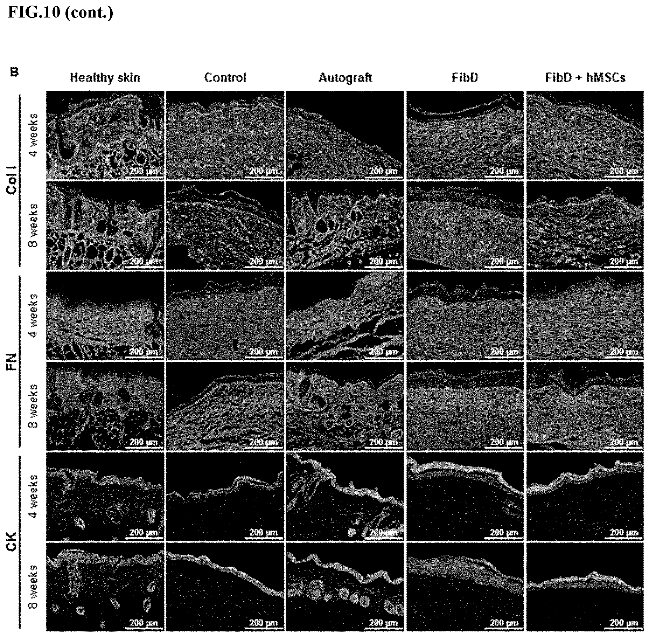

Figure 10: (A) H&E and MT histological staining; and (B) Col I, FN and CK immunofluorescence staining, of native and regenerated skin samples of mice after 4 and 8 weeks; scale bars: 200 $\mu$m.

## DEFINITIONS

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by $\pm 1, 2, 3, 4, 5, 6, 7, 8, 9,$ or 10%. Accordingly, the term "about" may mean the indicated value $\pm$ 5% of its value, preferably the indicated value $\pm$ 2% of its value, most preferably the term "about" means exactly the indicated value ($\pm$ 0%).

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

As used herein, the term "glycosaminoglycan" or "GAG" refers to a long, unbranched, polysaccharide molecules found on the cell surface or extracellular matrix. Non-limiting examples of glycosaminoglycan include heparin, chondroitin sulfate, dextran sulfate, dermatan sulfate, heparan sulfate, keratan sulfate, crosslinked or non-crosslinked hyaluronic acid, hexuronyl hexosaminoglycan sulfate, and inositol hexasulfate. Derivatives, salts and mimetics of the above, including low molecular weight heparin are intended to be included in the invention.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

The term "therapeutically effective amount" refers to an amount of matter which has a therapeutic effect, and which is able to treat the herein described diseases.

The terms "individual", "patient" or "subject" are used interchangeably in the present application and are not meant to be limiting in any way. The "individual", "patient" or "subject" can be of any age, sex and physical condition.

## DETAILED DESCRIPTION

The present invention may be understood more readily by reference to the following detailed description of the preferred embodiments of the invention, and to the examples included therein.

As described in more detail above, numerous advances have been developed in the past few decades for the management of cutaneous wounds, and the biofabrication of skin substitutes has gained special attention in recent years as an alternative to overcome the limitations of conventional skin autografting. Nevertheless, the manufacturing of some substitutes may be difficult, expensive, and time-consuming, sometimes needing several weeks for substitutes to be available. However, an early treatment of the wound is essential to achieve a better functional outcome, reduce scar formation, and avoid infections and related complications. The use of skin sprays offers a potential tool for wound healing applications, as they allow the delivery of different biomaterials and/or cell suspensions to the wound bed, being able to treat extensive wounds and even areas with unfavorable topography, rapidly and with great facility. Moreover, they avoid the need to come in contact with the wound, reducing pain for the patient. Even in cases of autologous cell sprays, the reduced donor area required for cell isolation shortens treatment time and re-epithelialization. Therefore, patients can be treated immediately in an outpatient setting, reducing their stay at the hospital, minimizing complications, and allowing to restore their normal mobility much faster. Despite their advantages, the translation of cell sprays to clinical practice is still limited and very few products have been approved for clinical use. There is therefore a need for providing novel bioinks for application in the treatment of skin lesions that are suitable for easy application, biocompatible and provide optimal wound healing properties.

These bioinks should on the one hand be suitable for use in sprays and on the other hand have the necessary mechanical properties for providing a suitable porosity and viscosity allowing cell migration and proliferation and the transport of nutrients and metabolic wastes, all of which prolongs cell viability and thus increases the healing efficacy. The bioinks should furthermore support the cell adherence on the damaged tissue for a prolonged time allowing the healing process to unfold.

The inventors have therefore herein developed novel bioinks for the treatment of tissues which can overcome the above disadvantages.

To this end two fibrinogen inks were formulated, one based on fibrinogen alone (Fib), and the other based on fibrinogen supplemented with GAGs/col matrix (FibD) as further detailed in Example 2. The ink is a source of GAGs/Col containing a high concentration of HA (67%), as well as sulfated GAGs (12%, including dermatan sulfate and chondroitin sulfate) and collagen (Col) (10.4%). These two inks were loaded with human mesenchymal stem cells (hMSCs) and human dermal fibroblasts (hDFs). The physicochemical properties of the bioinks, as well as their biocompatibility *in vitro,* were analyzed and found highly suitable for their intended application for spray application to the skin. Finally, their good skin wound healing properties were confirmed in an *in vivo* excisional wound healing murine model.

The present invention therefore relates in one aspect to a bioink formulation comprising fibrinogen and a glycosaminoglycans (GAGs)/collagen (Col) matrix, wherein the GAGs/Col matrix comprises at least

a) 60 - 75% (w/w) hyaluronic acid, and
b) 5 - 20% (w/w) sulphated GAGs, and
c) 1 - 5% (w/w) of collagen.

In one embodiment of the bioink formulation of present invention, the bioink formulation further comprises human cells, preferably human mesenchymal stem cells (hMSCs), keratinocytes and/or human dermal fibroblasts (hDFs).

In one embodiment of the bioink formulation of present invention the amount of fibrinogen in the formulation is between 1 and 100 mg/ml. In one embodiment of the bioink formulation of present invention the amount of fibrinogen in the formulation is between 0.1 and 10 % (w/v).

This amount of fibrinogen provides adequate viscosity and mechanical properties for the application, preferably as a spray, of the bioink onto the surface. Furthermore, the amount of fibrinogen allows, once mixed with thrombin, the formation of hydrogels in which the fibrin fibers form a matrix with a good density and porosity to allow the correct attachment, proliferation and viability of the cells.

In one embodiment the amount of fibrinogen in the formulation is between 2 and 90 mg/ml, between 3 and 80 mg/ml, between 4 and 70 mg/ml, between 5 and 60 mg/ml, between 6 and 50 mg/ml, between 7 and 40 mg/ml, between 8 and 30 mg/ml, between 9 and 20 mg/ml. In a preferred embodiment of the bioink formulation of present invention the amount of fibrinogen is about 10 mg/ml.

In one embodiment of the bioink formulation of present invention the amount of fibrinogen in the formulation is between 0.1 and 10% (w/v), between 0.2 and 9% (w/v), between 0.3 and 8% (w/v), between 0.4 and 7% (w/v), between 0.5 and 6% (w/v), between 0.6 and 5% (w/v), between 0.7 and 4% (w/v), between 0.8 and 3% (w/v), between 0.9 and 2% (w/v). In a preferred embodiment of the bioink formulation of present invention the amount of fibrinogen is about 1% (w/v).

In one embodiment of the bioink formulation of present invention the amount of GAGs/Col matrix in the formulation is between 1 and 25 mg/ml. In one embodiment of the bioink formulation of present invention the amount of GAGs/Col matrix in the formulation is between 0.1 and 2.5% (w/v).

This amount of GAGs/Col matrix provides for higher viability and number of cells. Furthermore, the GAGs/Col matrix provides better rheological properties to the bioink, as in terms of viscosity, the fibrinogen only bioink (Fib) presents a Newtonian behaviour, while the fibrinogen/ GAGs/Col matrix (FibD) bioink shows a shear-thinning behaviour.

In one embodiment of the bioink formulation of present invention the amount of GAGs/Col matrix in the formulation is between 1.1 and 20 mg/ml, between 1.2 and 15 mg/ml, between 1.3 and 10 mg/ml, between 1.4 and 5 mg/ml, between 1.5 and 4 mg/ml, between 1.6 and 3 mg/ml.

In a preferred embodiment the amount of GAGs/Col matrix in the formulation is 1.7 mg/ml. In an even more preferred embodiment, the amount of GAGs/Col matrix in the formulation is 2.5 mg/ml.

In one embodiment of the bioink formulation of present invention the amount of hyaluronic acid in the GAGs/Col matrix is between 65 and 70% (w/w), between 66 and 69% (w/w), between 67 and 68% (w/w). In a preferred embodiment the amount of hyaluronic acid in the GAGs/Col matrix is about 67% (w/w).

In one embodiment of the bioink formulation of present invention the amount of collagen in the GAGs/Col matrix is between 5 and 15% (w/w), preferably between 8 and 12% (w/w). In a preferred embodiment the amount of collagen in the GAGs/Col matrix is between 9 and 11% (w/w), most preferred about 10% (w/w).

In one embodiment of the bioink formulation of present invention the sulphated GAGs in the GAGs/Col matrix are selected from dermatan sulphate and/or chondroitin sulphate.

In one embodiment of the bioink formulation of present invention the amount of sulphated GAGs in the GAGs/Col matrix is between 10 and 15% (w/w), between 11 and 14% (w/w), preferably between 11 and 13% (w/w), most preferred about 12% (w/w).

In a further aspect the present invention relates to the bioink formulation as described herein for use in the treatment of tissue injuries or damages. In a preferred embodiment the tissue is skin.

In a further aspect the present invention relates to a method for treating diseased or injured tissue, the method comprising implanting/providing to the site of disease or injury a bioink formulation as described herein.

In one embodiment the tissue injuries or damages are skin injuries or damages, preferably selected from burns, wounds, chronic wounds, such as for example diabetic foot ulcers, pressure ulcers, venous leg ulcers, other skin injuries, such as psoriasis, dermatitis, acne, vitiligo.

In one embodiment, wherein the skin injury or damage is a wound, the method comprises a step of administering the bioink onto the wound during or after surgery for wound sealing.

In one embodiment of the bioink formulation for use in the treatment of tissue injuries or damages the step of administering the bioink formulation to said tissue induces hemostasis.

In another aspect the present invention relates to the use of the bioink formulation as described herein as a tissue adhesive or glue for surgery wounds/wound sealing.

In a further aspect the present invention relates to the use of the bioink of present invention for the treatment of bone injuries or for bone healing. In a preferred embodiment of this aspect the bioink formulation further comprises hydroxyapatite nanoparticles.

In a further aspect the present invention relates to the bioink formulation as described herein for use in the treatment of wrinkles or scars, or for the healing of the skin following aesthetic treatments, such as for example microneedling, laser, capacitive resistive monopolar radiofrequency, ultrasounds or peeling treatments.

The development and testing of the bioink of present invention is described herein below in further detail.

Cutaneous pH has an important role in the skin barrier function, maintaining the normal cutaneous microbiota, having antimicrobial functions, and influencing keratinization and desquamation. Human skin pH values reported in the literature are variable, ranging from 4.0 to 5.9, while other mammals present higher skin pH levels. As detailed in Example 3 a), the Fib and FibD bioinks of present invention presented acidic pH values of $6.12 \pm 0.19$ and $6.10 \pm 0.17$, respectively, adequate for their topical application. The swelling or liquid absorption ability of hydrogels influences the entrance of fluids and the transport of nutrients into the hydrophilic hydrogel matrices. The Fib and FibD hydrogels presented a satisfactory swelling capacity after just two days (Example 3 b)). The FibD hydrogels showed a higher swelling ratio in comparison to the Fib hydrogels, expected due to the presence of high concentrations of HA in the Dermial® component, given the high water retention capability of HA.

On the other hand, the biodegradability of the hydrogels is important as it affects cell growth and tissue regeneration. As detailed in Example 3 c) the Fib hydrogels degraded faster than FibD hydrogels, probably owing to the more complex matrix formed in the FibD hydrogels as a result of the different biomolecules of Dermial®. All Fib hydrogels had degraded completely after 17 weeks, whereas FibD hydrogels degraded after 20 weeks (i.e. Fib hydrogels degraded 15% faster than the FibD hydrogels).

In biofabrication, a high viscosity of the bioink or biomaterial ink is essential to maintain the 3D structure of the resulting construct, and specifically in skin sprays, to prevent run off of the sprayed material and prolong its contact time with the wound bed; but as mentioned previously, a higher viscosity of the transporting fluid affects cell viability negatively. Fibrinogen inks have been reported to exhibit a Newtonian fluid behavior, which is congruous with the results, where the Fib ink maintained a constant viscosity of $1.163 \pm 0.095$ mPa·s (Example 3 d)). On the other hand, the supplementation of the Fib ink with Dermial® resulted in a shear-thinning behavior of the FibD ink. This shear-thinning behavior is favorable for the use of bioinks in biofabrication techniques such as skin sprays or 3D bioprinting, given that the decrease in viscosity under high shear stress (during extrusion) reduces cell damage, and the subsequent recovery of high viscosity under low shear stress (after extrusion) helps to prevent leakage of the material and maintain the 3D shape.

Fibrin is one of the softest natural fibers, with a high elastic deformation capacity and large stretchability, but its mechanical properties can be optimized by adjusting the concentrations of fibrinogen and thrombin. Cell morphology, migration and protein expression of many cell types, including fibroblasts, are affected by the stiffness of their substrate. In fibrin matrices, the stiffness increases with higher concentrations of fibrinogen and of thrombin. In this study, fibrinogen and thrombin concentrations of 10 mg/mL and 50 U/mL, respectively, were used following manufacturer's recommendations. The Young's moduli of the hydrogels of present invention ranged between $0.169 \pm 0.025$ and $0.312 \pm 0.041$ kPa, falling within the reported 0.001 to 10 kPa range of substrate stiffness necessary for cell attachment. For all cellular hydrogels, the Young's Modulus was higher after 21 days compared to day 1, while the Storage and Loss moduli were lower at 21 days. This could be explained by the formation of ECM by cells within the fibrin matrix, and by the slightly shrinkage of the hydrogels observed as time progressed, due to cell proliferation, making hydrogels stiffer and less viscoelastic overtime. In acellular hydrogels, a slight reduction in stiffness and an increase in viscoelasticity could be observed in FibD hydrogels compared to Fib hydrogels. The reduction of fibrin stiffness when mixed with non-crosslinked HA has been previously reported. Moreover, the spraying process did not affect the stiffness and viscoelasticity of hydrogels significantly, in comparison to the pipetted controls.

Thereafter, the biocompatibility of the bioinks and hydrogels was analyzed *in vitro* with hDFs and hMSCs (Example 4 a)). Sprayed Fib and FibD hydrogels exhibited good proliferation rates with high viability levels through the 3 weeks of experiment for both hDFs and hMSCs, but the FibD hydrogels yielded better results in all cases. The same behavior was observed in the pipetted controls, with FibD hydrogels having higher cell proliferation and viability than Fib hydrogels; therefore demonstrating the beneficial effect of the addition of Dermial® on cells. However, there was a mild deceleration in cell proliferation after around one week in most cases, as the same time as a slight contraction of the hydrogels was observed. This shrinkage reduces the porosity of the fibrin matrices. There are reports of cell proliferation and viability being restrained in highly dense fibrin matrices, due to the reduction of the pore size, which would decrease the permeability to nutrients, and could subsequently lead to cell starvation in the inner core of the hydrogels. Like stiffness, porosity also depends on fibrinogen and thrombin concentrations. Nonetheless, the concentrations of fibrinogen and thrombin in the formulation of present invention were adequate to allow nutrients diffusion, and despite the slight shrinkage of gels, the viability and proliferation rates were still excellent after 3 weeks, especially in the FibD hydrogels. Similarly, it has been reported that although fibrinogen mixed with non-crosslinked HA resulted in a reduced porosity, the viability of hMSCs was not negatively affected thanks to the stimulation of cell attachment and differentiation by HA. On the other hand, in spite of sprayed hydrogels showing lower viability and proliferation rates than control hydrogels, expected due to the stresses caused during the spraying process, sprayed samples still maintained satisfactory levels of cell viability and proliferation, which over time recovered to similar levels than those of controls, revealing that these cell-loaded fibrin-based bioinks are suitable for their application with a spray system.

With respect to the scratch wound healing assay, the supplementation of the culture medium with Fib, Der, or a combination of both (Fib + Der) resulted in a faster healing of the scratch wound for both hDFs and hMSCs, in comparison to the non-treated controls (Example 4 b)). These results demonstrate that these biomaterials, fibrinogen and Dermial®, are beneficial to accelerate the wound healing of hDFs and hMSCs.

Wound healing is an intricate and complex process involving numerous cell types, chemokines and growth factors, consisting in four phases: hemostasis, inflammation, cell proliferation and remodeling. In this work, the healing process of full-thickness wounds in a murine model was studied, evaluating the closure rates, homeostasis parameters and histological structure of regenerated wounds treated with autografts or with our spray, in comparison to non-treated control wounds (Example 5). After the previous results, for the in vivo assay the inventors decided to use FibD (acellular) and FibD + hMSCs as spray treatments, given the advantages and beneficial effects of Dermial® on the mechanical and biological characteristics of the FibD hydrogels in comparison to the Fib ones. hMSCs were used due to their numerous advantages for the enhancement of tissue regeneration: by secreting paracrine factors, hMSCs are able to promote angiogenesis, regulate the function of other cell types involved in the wound healing process, attenuate scar formation, and reduce the inflammatory response. Moreover, their immunomodulatory properties allow them to be used as an allogeneic cellular treatment. The results revealed that mice treated with the FibD and FibD + hMSCs spray treatments presented the fastest wound closure, and that their wound repair was comparable to that of mice treated with autografts (the gold standard in clinical practice) and more effective than in non-treated mice, which showed the worst results; demonstrating the positive effect of the bioinks of present invention in the wound healing process.

An important difference between human and murine skin is their healing mechanisms: the former heals through re-epithelialization, while the latter heals through contraction. The inventors used a customized 3D printed b-TPUe splint in order to prevent wound contraction during the first few weeks, therefore ensuring that the observed wound closure was due to the different applied treatments and not wound contraction.

All groups of mice achieved an adequate recovery of the homeostasis parameters after 8 weeks, but the FibD and FibD + hMSCs groups yielded the best results, similar to those of Autograft, while the Control group had the biggest differences compared to healthy skin. The epidermal barrier plays a crucial role in thermoregulation. Temperature of regenerating wounds remained similar to that of healthy skin through the 8 weeks for all groups, indicating the correct restoration of this homeostatic function. The disruption of the skin barrier leads to an increase of pH, which was observed when comparing wounds to healthy skin after 2 weeks. As wounds healed over time, the pH decreased, and after 8 weeks, the difference in pH between wounds/scars and healthy skin was more statistically significant in Control mice than mice treated with Autograft, FibD and FibD + hMSCs. As well, when the integrity of the skin barrier is damaged, there is a higher TEWL, and consequently, a reduction of moisture. This was observed at week 2, where wounds presented TEWL levels much higher and moisture levels much lower than healthy skin; but after 4 weeks, wounds reached levels similar to healthy skin for both parameters in all groups. However, the FibD + hMSCs group was the one with the TEWL and moisture values with the smallest difference compared to healthy skin at week 2 (similar to the Autograft group for TEWL), and values almost identical to healthy skin from week 4 to 8. Given that in the Autograft group the wounds were covered with a biopsy of healthy skin, the mechanical properties, concretely the elasticity, of the regenerated wounds/scars of the Autograft group were the most similar to healthy skin during all the experiment, while the rest of the groups presented significant differences to healthy skin at week 2. After a skin injury, erythema (i.e., the redness of the skin) is caused by the hyperemia in superficial capillaries. The erythema levels of the wounds of the Autograft group were already similar to healthy skin at week 2, while the rest of the groups presented higher erythema levels at week 2, which progressively decreased until approaching healthy skin levels. The higher values of the FibD group at week 2 could be related to the angiogenic properties of fibrin and HA; but FibD + hMSCs was the group that presented the most similar results to Autograft. Pigmentation is a result of the melanin produced by melanocytes, which appear at the wound site once it has re-epithelialized. Therefore, hypopigmentation would be expected during the first weeks of the healing process. However, it is not atypical to observe hyperpigmentation of the wounds as they heal, caused by the activation of melanocytes by the inflammatory response during wound healing. This was observed especially in the case of the Control group, which had the highest melanin levels during all the experiment, whereas the Autograft, FibD and FibD + hMSCs groups had slightly lower levels, more similar to healthy skin, most likely due to the anti-inflammatory properties of the bioinks of present invention.

Finally, the histological and immunofluorescence staining revealed an appropriate regeneration of the healed wounds after 8 weeks (Example 7). The Autograft and FibD + hMSCs groups presented the best results compared to healthy skin after just 4 weeks, with a dense dermal matrix that expressed Col I (the major structural protein of the reticular dermis) and FN (the dominant glycoprotein in the provisional dermal matrix assembled for the formation of granulation tissue, with a crucial role in wound healing), and a thick epidermis with differentiated layers that expressed CK, which is a family of proteins that have been demonstrated to be markers of epidermal differentiation. On the other hand, non-treated Control wounds required longer time to achieve similar results.

All of these results demonstrate that even though all groups of mice were able to restore their skin barrier and functions correctly after 8 weeks, the spray treatment with the bioinks of present invention resulted in faster and better functional and aesthetic outcomes than non-treated mice, and although these results were comparable to those of Autograft, the spray treatment with these bioinks presents the advantages of avoiding the need to create a secondary wound to obtain an autologous biopsy, and allowing to have an allogeneic treatment that could be applied immediately instead of requiring weeks to become available.

In summary the inventors have shown that the bioinks of present invention are suitable for their spray application, forming hydrogels with satisfactory physicochemical, mechanical, and biological properties for their use as skin substitutes. The hydrogels yielded excellent cellular proliferation and viability and demonstrated in vivo that they can promote wound healing and tissue regeneration obtaining comparable results to those of autografts, while avoiding the downsides of the latter. Overall, these bioinks offer a promising therapeutic tool for the immediate treatment of skin wounds, enabling the use of either the acellular inks, or the bioinks loaded with autologous cells from the patient or with allogeneic cells such as hMSCs.

The following examples and figures are provided by way of illustration and are not intended to limit the scope of the present invention.

## EXAMPLES

### Example 1: Isolation of hMSCs and hDFs and cell culture

hMSCs and hDFs were isolated from human adipose tissue and skin samples, respectively, both collected at the Vithas Hospital Granada (Plastic Surgery Service) with informed consent and Institutional Review Board approval (ethic committee number: 0467-N-20), and characterized as previously reported. Both cell lines were cultured in high-glucose Dulbecco's Modified Eagle's Medium (DMEM; Sigma-Aldrich) supplemented with 10% fetal bovine serum (FBS) and 1% penicillin and streptomycin (P/S; Invitrogen). Cultures were maintained at 37°C in a humidified atmosphere containing 5% $CO2$. Medium was changed every 2-3 days, and cells were sub-cultured at approximately 80% confluence. For all the experiments, cells were used between passages 6 and 8.

### Example 2: Formulation of fibrinogen-based bioinks

Two different bioinks were formulated, one consisting of fibrinogen alone and another consisting of fibrinogen supplemented with Dermial®. To prepare the fibrinogen (Fib) ink, bovine fibrinogen (Sigma-Aldrich) was diluted in 0.9% NaCl at 37°C, to obtain a final fibrinogen concentration of 10 mg/mL. Once dissolved, the solution was filtered through 0.22 $\mu$m membrane filters (Merck Millipore) to ensure sterility. For the fibrinogen-Dermial® (FibD) ink, previously UV-sterilized Dermial® (Bioibérica S.A.U., Barcelona, Spain) was added to the Fib solution at a final concentration of 2.5 mg/mL. In case of preparing cell-loaded hydrogels, hMSCs or hDFs were then gently mixed with the Fib or FibD inks at a density of $1 \cdot 10^6$ cells/mL. As well, bovine thrombin (Sigma-Aldrich) was diluted in 40mM $CaCl_2$ at a final concentration of 50 U/mL. Finally, the fibrin-based hydrogels were obtained by mixing the Fib or FibD bioinks with the thrombin solution in a 10:1 (v/v) ratio, respectively, and letting them gel for at least 5 minutes at 37°C.

### Example 3: Determination of Physicochemical properties

a) pH determination

The pH of the Fib and FibD inks was measured (n=6) with a calibrated digital pH-meter Hach Sension+ (Hach Lange S.L., Spain) at room temperature. The pH values of the Fib and FibD inks were 6.12 ± 0.19 and 6.10 ± 0.17, respectively.

b) Swelling test of Fib and FibD hydrogels

Previously freeze-dried hydrogels were weighed, submerged in PBS, and kept at 37°C. At determined time points, samples (n=6) were taken out, absorbing excess of PBS with filter paper, and weighed. The swelling rates (%) were calculated as a measure of weight gain with the following equation:

$$Swelling\ ratio\ (\%) = \left(\frac{W_t - W_0}{W_0}\right) \times 100 \qquad (1)$$

$W_0$ is the initial wet weight of samples (t = 0 $h$).

$W_t$ is the wet weight of samples at the corresponding time point (t).

The swelling behavior of previously freeze-dried Fib and FibD hydrogels was observed by immersing the hydrogels in PBS and measuring weight increase (Figure 2A). For both Fib and FibD hydrogels, the swelling ratio increased during the first 48h, reaching an average swelling of 66.7 $\pm$ 10.2 % and 72.2 $\pm$ 5.0 % for Fib and FibD, respectively. Afterwards, hydrogels' weight started decreasing slightly due to degradation. The swelling ratio of the FibD hydrogels was higher than Fib at all time points, but no significant differences were observed.

c) Degradation test of Fib and FibD hydrogels

Pre-weighed hydrogels were immersed in PBS and maintained at 37°C under gentle agitation. At determined time intervals, samples (n=6) were centrifuged at 3000 G for 10 minutes, the supernatant was removed, and hydrogels were weighed. The degradation rates (%) as a measure of weight loss were calculated with the following equation:

$$Degradation\ ratio\ (\%) = \left(\frac{W_0 - W_t}{W_0}\right) \times 100 \qquad (2)$$

$W_0$ is the initial wet weight of samples (t = 0 *h).*
$W_t$ is the wet weight of samples at the corresponding time point (t).

The degradation rate of hydrogels was analyzed by measuring weight loss over time (Figure 2B). The degradation of Fib and FibD hydrogels increased rapidly during the first week, and then kept increasing moderately every week. The Fib hydrogels degraded slightly faster than the FibD hydrogels, presenting a higher degradation ratio during all the experiment, with significant differences at 6 h, days 5 and 7, and weeks 5 to 12. All Fib hydrogels had degraded completely after 17 weeks, whereas FibD hydrogels degraded after 20 weeks (i.e. Fib hydrogels degraded 15% faster than the FibD hydrogels).

D) Mechanical and rheological characterization

Rheological assays were carried out in order to obtain information on the viscosity of the fibrin-based inks, as well as on the compression and shearing characteristics of the hydrogels, using a torsional rheometer MCR302 (Anton Paar, Austria). All the rheological assays were performed in triplicate for all conditions and at a temperature of 25°C.

The shear viscosity was obtained using a cone-plate geometry (50 mm diameter and 1° angle). Samples of each material (distilled water, NaCl 0.9%, and the Fib and FibD inks) were sequentially placed on the base of the rheometer. First, they were rotatory pre-sheared at a constant shear rate of 800 s$^{-1}$ for 1 min. Then, they were allowed to rest for 1 min, with no shear rate applied. Lastly, they were rotatory sheared with a logarithmic shear rate ramp from 0.01 to 800 s$^{-1}$ (acquisition time of 5 seconds) for 5 min.

A plate-plate geometry (20 mm diameter and 5 mm gap) was used to analyze the compression and oscillatory shear behavior of hydrogels (prepared with a diameter of 20 mm and a height of approximately 5 mm) and mice skin samples (cut in a circular form with the same diameter, 20 mm). First, samples were placed onto the base of the rheometer and were approached by the rheometer head at a constant speed of 10 $\mu$m/s, up to a normal force of 0.1 N (in the case of the hydrogels) or 0.5 N (in the case of the mice skin), to determine the Young's modulus. Secondly, the normal force was kept constant for 30 seconds at 0.1 N or 0.5 N, correspondingly. Finally, to determine the shear viscoelastic moduli, the samples were oscillatory sheared according to a strain amplitude of 0.001% to 1000% at a frequency of 1 Hz and a normal force of 0.1 N (for hydrogels) or 0.5 N (for mice skin).

In order to assess the capability of the inks to be sprayed through the airbrush's nozzle, the shear viscosity of the Fib and FibD inks, as well as distilled water and 0.9% NaCl (the basis of the fibrin inks formulation), was determined with a rotational shear test (Figure 2C). At 25°C, water and NaCl obtained viscosity results of 0.871 $\pm$ 0.039 and 0.896 $\pm$ 0.272 mPa·s, respectively. As expected, they demonstrated a Newtonian flow behavior, with viscosity being independent of the shear rate. Interestingly, despite being a polymeric solution, the Fib ink also showed a Newtonian behavior, with a viscosity of 1.163 $\pm$ 0.095 mPa·s. However, the addition of Dermial® (which contains high concentrations of HA molecules) resulted in the FibD ink having a shear-thinning behavior, with a decreasing viscosity with increasing shear rates (ranging from 1,114.75 mPa·s at $\gamma$ ˙=0.1 s-1, to 2.97 mPa·s at $\gamma$ ˙=800 s-1), typical of polymeric solutions.

On the other hand, the compression and oscillatory shearing characteristics of the hydrogels formed from the bioinks are analyzed in Figures 2D, E and F. Manually created (pipetted) Fib and FibD hydrogels (Control) were compared with sprayed Fib and FibD hydrogels, respectively, with no significant differences found. Between Fib and FibD hydrogels, differences were not statistically significant, but higher Storage (Figure 2E) and Loss (Figure 2F) moduli could be observed for the FibD hydrogels than the Fib ones, but this difference was not observable in cell-loaded hydrogels. There were significant differences in the Young's Modulus between sprayed acellular FibD hydrogels and sprayed hDFs-loaded FibD hydrogels, both after 1 and 21 days (Figure 2D); as well as in the Storage and Loss moduli between sprayed acellular Fib hydrogels and sprayed hDFs-loaded Fib hydrogels after 21 days in culture; and between sprayed acellular FibD hydrogels and hDFs-loaded FibD hydrogels after 21 days (Figures 2E and F). For hDFs and hMSCs-loaded hydrogels, both with Fib and FibD, differences were observed when comparing hydrogels maintained in culture for 1 or 21 days: hydrogels cultured for 21 days showed higher Young's moduli (Figure 2D) and lower Viscoelastic moduli (Figures 2E and F), than those cultured for 1 day. Finally, samples of healthy skin of mice revealed higher Young's and Viscoelastic moduli than all hydrogels, but the difference was not statistically significant for the Young's Modulus.

## Example 4: Determination of Biological properties

a) Cell proliferation and viability

Cell proliferation, related to metabolic activity, was analyzed with the AlamarBlue HS® assay after 0, 1, 3, 5, 7, 14, and 21 days of culture, as described previously.

The LIVE/DEAD™ Viability/Cytotoxicity Kit (Invitrogen Inc., Grand Island, NY, USA) was used to assess cell viability after 1, 7, 14, and 21 days of culture. Samples were stained with a calcein AM (2 $\mu$M) and ethidium homodimer I (EthD-I; 4 $\mu$M) solution in PBS at 37°C for 30 minutes. Samples were visualized with confocal microscopy (Leica TCS-SP5 II DMI6000B, Leica Microsystems), showing viable cells green (calcein AM) and non-viable cells red (EthD-I), with laser setting at 494/517 nm for calcein AM and 528/617 nm for EthD-I. Images were analyzed with the imaged (Fiji) software, quantifying live and dead cells, and determining the percentage of cell viability with the following equation:

$$Cell\ viability\ (\%) = \left(\frac{Live\ cells}{Live + dead\ cells}\right) \times 100 \qquad (3)$$

The biocompatibility of the Fib and FibD bioinks was analyzed with cell proliferation and viability assays. Hydrogels were created by simultaneously spraying the thrombin solution and the Fib and FibD bioinks loaded with hDFs or hMSCs. On the other hand, hydrogels created manually were used as controls.

Cell viability and proliferation of sprayed hydrogels are shown in Figure 3. During the duration of the assay, hDFs showed cell viability rates between 84.3 - 96.4% in the Fib hydrogels and between 92.1 - 97.6% in the FibD hydrogels, whereas hMSCs had viability rates between 87.3 - 95.2% in the Fib hydrogels and between 91 - 96% in the FibD hydrogels. For both hDFs and hMSCs, viability was slightly higher in the FibD hydrogels than in the Fib hydrogels for each day, although only statistically significant for the hDFs at days 1 and 21. After 21 days, there was a mild reduction in hMSCs viability in the Fib hydrogels, but not in the FibD hydrogels (Figure 3B). This was also evidenced by the confocal images shown in Figure 3A, with live cells (in green) seemingly more numerous and elongated in FibD hydrogels, and with fewer dead cells (in red) than in the Fib hydrogels. Similarly, the proliferation rate for both hDFs and hMSCs was well maintained through 3 weeks, with maximum peaks after around 3-7 days, which then started decreasing moderately and stabilized after between 14 and 21 days. Nonetheless, hDFs and hMSCs proliferation was higher in the FibD hydrogels than in the Fib ones during all the experiment (Figure 3C).

With respect to the control hydrogels (Figure 4), cells showed a similar behavior to the sprayed hydrogels, with both hDFs and hMSCs showing a higher proliferation and viability in the FibD hydrogels than in the Fib ones in all cases. Moreover, when comparing sprayed hydrogels to the pipetted controls (Figure 5), the latter showed a higher viability and proliferation than sprayed hydrogels for both hDFs and hMSCs. Despite this, sprayed samples still maintained high proliferation ratios and excellent viability, comparable to those of pipetted controls.

b) Scratch wound healing assay

A scratch assay was carried out to assess the potential of the biomaterials used for the fibrin-based bioinks to promote wound healing. Figure 6A shows representative microscope images of wounds over time, and the % of wound closure, measured as the reduction in the wound areas compared to 0 hours, is represented in Figure 6B. For both hDFs and hMSCs, wounds treated with culture media supplemented with Fib, Der, and Fib + Der showed a higher wound closure percentage than controls (wounds treated with normal culture medium) after 12 and 24 hours. Furthermore, hDFs wounds treated with Der and with Fib + Der, as well as hMSCs wounds treated with Fib + Der, had reached a wound closure close to 100% after 24 hours, displaying a faster wound healing rate than the control group. All wounds were closed after 48 hours.

**Example 5: *In vivo* wound healing assay**

The surgical procedure was well tolerated by all animals, without complications. All groups of mice achieved an appropriate wound stabilization after 4 weeks. Mice treated with Autograft, FibD and FibD + hMSCs presented a slightly faster wound repair than Control mice, which showed a slower wound closure. After 8 weeks, skin regeneration was more effective for the FibD and FibD + hMSCs groups, while the Control group had a worse wound repair (Figure 5A).

Visual evaluation of scars was further assessed using an adaptation of the POSAS scale (Table 1), where the total score revealed that mice treated with FibD and with FibD + hMSCs presented comparable results to mice treated with Autograft, while the Control group showed the worst results.

Table 1. POSAS scale results after 8 weeks.

| | Vascularity[1] | Pigmentation[1] | Thickness[1] | Relief[1] | Pliability[1] | Surface area[1] | Total score[2] |
|---|---|---|---|---|---|---|---|
| Control | 4 | 4 | 3 | 2 | 1 | 4 | 18 |
| Autograft | 3 | 2 | 2 | 2 | 1 | 3 | 13 |
| FibD | 3 | 2 | 3 | 2 | 1 | 3 | 14 |
| FibD + hMSCs | 3 | 2 | 3 | 2 | 1 | 2 | 13 |
| [1]All items are scored on a scale ranging from 1 ('like normal skin') to 10 ('worst imaginable scar').<br>[2]The lowest score, 6, reflects normal skin, whereas the highest score, 60, reflects the worst imaginable scar. | | | | | | | |

Similarly, results by visual clinical evaluation (Figure 7A) correlated with the quantitative analysis of the wound/scar area (Figure 7B), where significant differences were found in the wound/scar area between the Autograft and Control groups at 28 days, and between the FibD and FibD + hMSCs groups when compared with Control at 42 and 56 days. Moreover, differences in wound closure at day 56 compared to day 14 were more statistically significant for the FibD and FibD + hMSCs groups than the Autograft and Control groups. These results indicate a positive effect of the FibD and FibD + hMSCs treatments in accelerating the wound healing process.

**Example 6: Homeostasis analysis**

Several homeostasis parameters were monitored through 8 weeks, comparing the regenerated wounds or scars to areas of healthy skin for each group of mice (Control, Autograft, FibD, or FibD + hMSCs) (Figure 8). For all groups, temperature values (Figures 6A, H, O and V) remained similar to those of healthy skin during all the experiment, with no significant differences found after 8 weeks. On the contrary, pH of wounds/scars decreased below healthy skin values after 4 weeks for all groups (Figures 6B, I, P and W), but with the Control group having the most significant difference between scars and healthy skin after 8 weeks (Figure 8B). At week 2, Autograft and FibD + hMSCs were the groups with TEWL levels closer to healthy skin values; but then TEWL decreased significantly after 2 weeks, reaching healthy skin levels for all groups (Figures 8C, J, Q and X). Regarding elasticity (Figures 8D, K, R and Y), Autograft was the only group that showed no significant differences in wounds/scars elasticity compared to healthy skin through the 8 weeks, while Control, FibD and FibD + hMSCs groups had significant differences at week 2, and FibD also at week 8. For all groups, moisture values of wounds/scars were significantly much lower than those of healthy skin at week 2, but they recovered to healthy skin values after 4 weeks, with the FibD + hMSCs group showing identical values to healthy skin (Figures 8E, L, S and Z). Finally, erythema (Figures 8F, M, T and AA) and melanin (Figures 8G, N, U and AB) showed a similar tendency for all groups: starting with wound/scar values being significantly higher than healthy skin, and decreasing through the weeks until achieving healthy skin levels after 6-8 weeks; except for the Autograft group, whose erythema values at week 2 were already similar to healthy skin. Figure 9 shows, for each parameter, the comparison of the different groups against the mean values of healthy skin of all groups.

**Example 7: Histology and immunofluorescence**

The histological staining with H&E and MT depicted an adequate regeneration of the dermis and epidermis 4 and 8 weeks after the surgical procedure for all groups (Figure 10A). After 4 weeks, the Autograft and FibD + hMSCs groups showed a thicker epidermis, where the different layers (stratum basale, spinosum, granulosum and corneum) could be differentiated, while the epidermis of the Control group did not show differentiated layers; but after 8 weeks, all groups reached similar results. The Autograft, FibD and FibD + hMSCs groups presented a denser dermal matrix after 4 weeks, whereas the Control group's dermis was not as dense until after 8 weeks. As well, Autograft and FibD + hMSCs were the groups in which the papillary and reticular dermis could be slightly distinguishable after 4 weeks. Skin appendages such as sebaceous and sweat glands, present in healthy skin, could not be observed in the regenerated wounds of any group.

With respect to the immunofluorescence staining (Figure 10B), the expression of Col I and FN was observed in all groups after 4 and 8 weeks, showing a good regeneration of the dermis. The regeneration of the epidermis in all groups was evidenced by the expression of CK. However, skin appendages were only present in the Autograft group.

These results indicate that the Autograft and FibD + hMSCs treatments are capable of achieving the regenerated dermis and epidermis most similar to native healthy skin after just 4 weeks, followed by the FibD treatment, with Control (no treatment) needing more time to obtain similar results.

## References

[1] TISSEEL, (n.d.). https://globaladvancedsurgery.baxter.com/tisseel (accessed October 18, 2022).

[2] ARTISS, (n.d.). https://globaladvancedsurgery.baxter.com/artiss (accessed October 18, 2022).

[3] VISTASEAL™ Fibrin Glue & Sealant | Ethicon, (n.d.). https://www.jnjmedtech.com/en-US/product/vistaseal-fibrin-sealant-human (accessed October 18, 2022).

[4] Vivostat - Fibrin Sealant, (n.d.). https://vivostat.com/products/vivostat-fibrin-sealant (accessed October 18, 2022).

[5] O. Catanzano, M.C. Straccia, A. Miro, F. Ungaro, I. Romano, G. Mazzarella, G. Santagata, F. Quaglia, P. Laurienzo, M. Malinconico, Spray-by-spray in situ crosslinking alginate hydrogels delivering a tea tree oil microemulsion, European Journal of Pharmaceutical Sciences. 66 (2015) 20-28. https://doi.org/10.1016/j.ejps.2014.09.018.

[6] P.M. Neumann, B. Zur, Y. Ehrenreich, Gelatin-based sprayable foam as a skin substitute, J Biomed Mater Res. 15 (1981) 9-18. https://doi.org/10.1002/jbm.820150105.

[7] Y. Li, Q.Q. Leng, X.L. Pang, H. Shi, Y.L. Liu, S.S. Xiao, L. Zhao, P. Zhou, S.Z. Fu, Therapeutic effects of EGF-modified curcumin/chitosan nano-spray on wound healing, Regen Biomater. 8 (2021). https://doi.org/10.1093/RB/RBAB009.

[8] J. Chen, H. Wang, L. Mei, B. Wang, Y. Huang, G. Quan, C. Lu, T. Peng, X. Pan, C. Wu, A pirfenidone loaded spray dressing based on lyotropic liquid crystals for deep partial thickness burn treatment: healing promotion and scar prophylaxis, J Mater Chem B. 8 (2020) 2573-2588. https://doi.org/10.1039/C9TB02929J.

[9] K.M.G. Jáuregui, J.C.C. Cabrera, E.P.S. Ceniceros, J.L.M. Hernández, A. Ilyina, A new formulated stable papin-pectin aerosol spray for skin wound healing, Biotechnology and Bioprocess Engineering. 14 (2009) 450-456. https://doi.org/10.1007/s12257-008-0268-0.

[10] B. ter Horst, R.J.A. Moakes, G. Chouhan, R.L. Williams, N.S. Moiemen, L.M. Grover, A gellan-based fluid gel carrier to enhance topical spray delivery, Acta Biomater. 89 (2019) 166-179. https://doi.org/10.1016/j.actbio.2019.03.036.

[11] W.S. Veazey, K.U. Anusavice, K. Moore, Mammalian cell delivery via aerosol deposition, J Biomed Mater Res B Appl Biomater. 72 (2005) 334-338. https://doi.org/10.1002/jbm.b.30159.

[12] F.A. Navarro, M.L. Stoner, C.S. Park, J.C. Huertas, H.B. Lee, F.M. Wood, D.P. Orgill, Sprayed Keratinocyte Suspensions Accelerate Epidermal Coverage in a Porcine Microwound Model, Journal of Burn Care & Rehabilitation. 21 (2000) 513-518. https://doi.org/10.1097/00004630-200021060-00007.

[13] F.O.G. Fraulin, A. Bahoric, A.R. Harrop, T. Hiruki, H.M. Clarke, Autotransplantation of epithelial cells in the pig via an aerosol vehicle, Journal of Burn Care and Rehabilitation. 19 (1998) 337-345. https://doi.org/10.1097/00004630-199807000-00012.

[14] J.C. Gerlach, C. Johnen, C. Ottoman, K. Bräutigam, J. Plettig, C. Belfekroun, S. Münch, B. Hartmann, Method for autologous single skin cell isolation for regenerative cell spray transplantation with non-cultured cells, International Journal of Artificial Organs. 34 (2011) 271-279. https://doi.org/10.5301/IJAO.2011.6508.

[15] B. Hartmann, A. Ekkernkamp, C. Johnen, J.C. Gerlach, C. Belfekroun, M. v. Küntscher, Sprayed Cultured Epithelial Autografts for Deep Dermal Burns of the Face and Neck, Ann Plast Surg. 58 (2007) 70-73. https://doi.org/10.1097/01.sap.0000250647.39784.bb.

[16] P. Johnstone, J.S.S. Kwei, G. Filobbos, D. Lewis, S. Jeffery, Successful application of keratinocyte suspension using autologous fibrin spray, Burns. 43 (2017) e27-e30. https://doi.org/10.1016/j.burns.2016.05.010.

[17] R. Esteban-Vives, M.S. Choi, M.T. Young, P. Over, J. Ziembicki, A. Corcos, J.C. Gerlach, Second-degree burns with six etiologies treated with autologous noncultured cell-spray grafting, Burns. 42 (2016) e99-e106. https://doi.org/10.1016/j.burns.2016.02.020.

[18] AVITA MEDICAL, (n.d.). https://avitamedical.com/product/ (accessed October 13, 2022).

[19] Our Technology | RenovaCare, (n.d.). https://www.renovacareinc.com/technology/ (accessed October 17, 2022).

**Claims**

1. A bioink formulation comprising fibrinogen and a glycosaminoglycans (GAGs)/collagen (Col) matrix, wherein the GAGs/Col matrix comprises at least

   a) 60 - 75% (w/w) hyaluronic acid, and
   b) 5 - 20% (w/w) sulphated GAGs, and
   c) 1 - 5% (w/w) of collagen.

2. The bioink formulation of claim 1 further comprising human cells, preferably human mesenchymal stem cells (hMSCs), keratinocytes and/or human dermal fibroblasts (hDFs).

3. The bioink formulation of claims 1 or 2, wherein the amount of fibrinogen in the formulation is

   (i) between 1 and 100 mg/ml, preferably about 10 mg/ml, and/or
   (ii) between 0.1 and 10 % (w/v), preferably about 1% (w/v).

4. The bioink formulation of any one of the preceding claims, wherein the amount of GAGs/Col matrix in the formulation is

   (i) between 1 and 25 mg/ml, and/or
   (ii) between 0.1 and 2.5% (w/v).

5. The bioink formulation of any one of the preceding claims, wherein the amount of hyaluronic acid in the GAGs/Col matrix is between 65 and 70% (w/w).

6. The bioink formulation of any one of the preceding claims, wherein the amount of collagen in the GAGs/Col matrix is between 5 and 15% (w/w), preferably between 8 and 12% (w/w).

7. The bioink formulation of any one of the preceding claims, wherein the sulphated GAGs are selected from dermatan sulfate and/or chondroitin sulfate.

8. The bioink formulation of any one of the preceding claims, wherein the amount of sulphated GAGs in the GAGs/Col matrix is between 10 and 15% (w/w).

9. The bioink formulation of any one of the preceding claims for use in the treatment of tissue injuries or damages comprising a step of administering the bioink formulation to the tissue.

10. The bioink formulation for use in the treatment of tissue injuries or damages according to claim 9, wherein the tissue is skin.

11. The bioink formulation of any one of the preceding claims for use in the treatment of tissue injuries or damages according to claim 9, wherein the tissue injuries or damages are skin injuries or damages, preferably selected from burns, wounds, chronic wounds, such as for example diabetic foot ulcers, pressure ulcers, venous leg ulcers, other skin injuries, such as psoriasis, dermatitis, acne, vitiligo.

12. The bioink formulation of any one of the preceding claims for use in the treatment of wrinkles or scars, or for the healing of the skin following aesthetic treatments, such as for example microneedling, laser, capacitive resistive monopolar radiofrequency, ultrasounds or peeling treatments.

13. The bioink formulation of any one of the preceding claims for use in the treatment of tissue injuries or damages according to claims 9 to 11, wherein the step of administering the bioink formulation to said tissue induces hemostasis.

14. The bioink formulation of any one of claims 1 to 8 for use in the treatment of bone injuries or damages.

15. The bioink formulation of any one of claims 1 to 8 for use in the treatment of bone injuries or damages according to claim 14, wherein the bioink formulation further comprises hydroxyapatite nanoparticles.

**FIG.1**

**FIG.2**

**FIG.3**

FIG.3 (cont.)

B  Cell viability

C  Cell proliferation

hDFs + Fib
hDFs + FibD
hMSCs + Fib
hMSCs + FibD

**FIG.4**

FIG. 5

EP 4 631 538 A1

**FIG.6**

A

hDFs

hMSCs

FIG.6 (cont.)

**FIG.7**

**FIG.8**

**FIG.9**

**FIG.10**

**FIG.10 (cont.)**

Europäisches
Patentamt
European
Patent Office
Office européen
des brevets

# EUROPEAN SEARCH REPORT

**Application Number**

EP 24 38 2364

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2023/372575 A1 (MARCHAL CORRALES JUAN ANTONIO [ES] ET AL) 23 November 2023 (2023-11-23) * paragraphs 112, 258-261 * | 1-15 | INV. A61L27/26 A61L27/38 A61L27/50 A61L27/58 B33Y70/00 B33Y80/00 |
| A | DE MELO BRUNA A G ET AL: "Strategies to use fibrinogen as bioink for 3D bioprinting fibrin-based soft and hard tissues", ACTA BIOMATERIALIA, ELSEVIER, AMSTERDAM, NL, vol. 117, 16 September 2020 (2020-09-16), pages 60-76, XP086330230, ISSN: 1742-7061, DOI: 10.1016/J.ACTBIO.2020.09.024 [retrieved on 2020-09-16] * Abstract and table 1 * | 1-15 | |
| Y | JORGENSEN ADAM M ET AL: "Decellularized Skin Extracellular Matrix (dsECM) Improves the Physical and Biological Properties of Fibrinogen Hydrogel for Skin Bioprinting Applications", NANOMATERIALS, vol. 10, no. 8, 29 July 2020 (2020-07-29), page 1484, XP093206038, ISSN: 2079-4991, DOI: 10.3390/nano10081484 * Abstract; sections 2.1-2.3 and 3 * | 1-15 | **TECHNICAL FIELDS SEARCHED (IPC)** A61L B33Y |

The present search report has been drawn up for all claims

| Place of search | Date of completion of the search | Examiner |
|---|---|---|
| The Hague | 18 September 2024 | Cadamuro, Sergio |

EPO FORM 1503 03.82 (P04C01)

## ANNEX TO THE EUROPEAN SEARCH REPORT
## ON EUROPEAN PATENT APPLICATION NO.

EP 24 38 2364

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2024

| Patent document cited in search report | Publication date | Patent family member(s) | Publication date |
|---|---|---|---|
| US 2023372575 A1 | 23-11-2023 | EP 4279093 A1 | 22-11-2023 |
| | | ES 2956802 A1 | 28-12-2023 |
| | | US 2023372575 A1 | 23-11-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021015631 A
- US 2006172008 A1

- US 2017304600 A1

**Non-patent literature cited in the description**

- **O. CATANZANO** ; **M.C. STRACCIA** ; **A. MIRO** ; **F. UNGARO** ; **I. ROMANO** ; **G. MAZZARELLA** ; **G. SANTAGATA** ; **F. QUAGLIA** ; **P. LAURIENZO** ; **M. MALINCONICO**. Spray-by-spray in situ crosslinking alginate hydrogels delivering a tea tree oil microemulsion. *European Journal of Pharmaceutical Sciences*, 2015, vol. 66, 20-28, https://doi.org/10.1016/j.ejps.2014.09.018
- **P.M. NEUMANN** ; **B. ZUR** ; **Y. EHRENREICH**. Gelatin-based sprayable foam as a skin substitute. *J Biomed Mater Res.*, 1981, vol. 15, 9-18, https://doi.org/10.1002/jbm.820150105
- **Y. LI** ; **Q.Q. LENG** ; **X.L. PANG** ; **H. SHI** ; **Y.L. LIU** ; **S.S. XIAO** ; **L. ZHAO** ; **P. ZHOU** ; **S.Z. FU**. Therapeutic effects of EGF-modified curcumin/chitosan nanospray on wound healing. *Regen Biomater.*, 2021, vol. 8, https://doi.org/10.1093/RB/RBAB009
- **J. CHEN** ; **H. WANG** ; **L. MEI** ; **B. WANG** ; **Y. HUANG** ; **G. QUAN** ; **C. LU** ; **T. PENG** ; **X. PAN** ; **C. WU**. A pirfenidone loaded spray dressing based on lyotropic liquid crystals for deep partial thickness burn treatment: healing promotion and scar prophylaxis. *J Mater Chem B.*, 2020, vol. 8, 2573-2588, https://doi.org/10.1039/C9TB02929J
- **K.M.G. JÁUREGUI** ; **J.C.C. CABRERA** ; **E.P.S. CENICEROS** ; **J.L.M. HERNÁNDEZ** ; **A. ILYINA**. A new formulated stable papin-pectin aerosol spray for skin wound healing. *Biotechnology and Bioprocess Engineering*, 2009, vol. 14, 450-456, https://doi.org/10.1007/s12257-008-0268-0
- **B. TER HORST** ; **R.J.A. MOAKES** ; **G. CHOUHAN** ; **R.L. WILLIAMS** ; **N.S. MOIEMEN** ; **L.M. GROVER**. A gellan-based fluid gel carrier to enhance topical spray delivery. *Acta Biomater.*, 2019, vol. 89, 166-179, https://doi.org/10.1016/j.actbio.2019.03.036
- **W.S. VEAZEY** ; **K.U. ANUSAVICE** ; **K. MOORE**. Mammalian cell delivery via aerosol deposition. *J Biomed Mater Res B Appl Biomater.*, 2005, vol. 72, 334-338, https://doi.org/10.1002/jbm.b.30159

- **F.A. NAVARRO** ; **M.L. STONER** ; **C.S. PARK** ; **J.C. HUERTAS** ; **H.B. LEE** ; **F.M. WOOD** ; **D.P. ORGILL**. Sprayed Keratinocyte Suspensions Accelerate Epidermal Coverage in a Porcine Microwound Model. *Journal of Burn Care & Rehabilitation*, 2000, vol. 21, 513-518, https://doi.org/10.1097/00004630-200021060-00007
- **F.O.G. FRAULIN** ; **A. BAHORIC** ; **A.R. HARROP** ; **T. HIRUKI** ; **H.M. CLARKE**. Autotransplantation of epithelial cells in the pig via an aerosol vehicle. *Journal of Burn Care and Rehabilitation*, 1998, vol. 19, 337-345, https://doi.org/10.1097/00004630-199807000-00012
- **J.C. GERLACH** ; **C. JOHNEN** ; **C. OTTOMAN** ; **K. BRÄUTIGAM** ; **J. PLETTIG** ; **C. BELFEKROUN** ; **S. MÜNCH** ; **B. HARTMANN**. Method for autologous single skin cell isolation for regenerative cell spray transplantation with non-cultured cells. *International Journal of Artificial Organs*, 2011, vol. 34, 271-279, https://doi.org/10.5301/IJAO.2011.6508
- **B. HARTMANN** ; **A. EKKERNKAMP** ; **C. JOHNEN** ; **J.C. GERLACH** ; **C. BELFEKROUN** ; **M. V. KÜNTSCHER**. Sprayed Cultured Epithelial Autografts for Deep Dermal Burns of the Face and Neck. *Ann Plast Surg.*, 2007, vol. 58, 70-73, https://doi.org/10.1097/01.sap.0000250647.39784.bb
- **P. JOHNSTONE** ; **J.S.S. KWEI** ; **G. FILOBBOS** ; **D. LEWIS** ; **S. JEFFERY**. Successful application of keratinocyte suspension using autologous fibrin spray. *Burns*, 2017, vol. 43, e27-e30, https://doi.org/10.1016/j.burns.2016.05.010
- **R. ESTEBAN-VIVES** ; **M.S. CHOI** ; **M.T. YOUNG** ; **P. OVER** ; **J. ZIEMBICKI** ; **A. CORCOS** ; **J.C. GERLACH**. Second-degree burns with six etiologies treated with autologous noncultured cell-spray grafting. *Burns*, 2016, vol. 42, e99-e106, https://doi.org/10.1016/j.burns.2016.02.020